# EUROPÄISCHE PATENTANMELDUNG

(11) **EP 1 762 220 A2**
(43) Veröffentlichungstag der Anmeldung: **14.03.2007**
(21) Anmeldenummer: 06017848.0
(22) Anmeldetag: 26.08.2006
(51) Int. Cl.: A61K 8/49, A61K 8/81, A61Q 5/10

(54) **Mittel zur Färbung keratinischer Fasern enthaltend Polymere mit kleiner Molmasse**

(30) Priorität: 09.09.2005 DE 102005043187
(71) Anmelder: Henkel Kommanditgesellschaft auf Aktien, 40589 Düsseldorf (DE)
(72) Erfinder: Kleen, Astrid, 22763 Hamburg (DE); Frauendorf, Bianca, 42549 Velbert (DE)

(57) **Zusammenfassung**

Die vorliegende Erfindung betrifft Mittel zur Farb- und/oder dauerhaften Formveränderung keratinischer Fasern, enthaltend in einem kosmetisch akzeptablen Träger mindestens einen Wirkstoff, der die Farbe und/oder dauerhaft die Form der keratinischen Fasern verändert, die weiterhin
(A) mindestens ein Alkyl- und/oder Alkenyl-Oligoglykosid und/oder eines seiner Derivate und
(B) mindestens ein kationisches und/oder amphoteres Polymer mit einer mittleren molaren Masse unterhalb von 20.000
enthalten.

## Beschreibung

Die vorliegende Erfindung betrifft ein Mittel zur Färbung keratinischer Fasern, das ein Alkyl- und/oder Alkenyloligoglukosid und ein kationisches und/oder amphoteres Polymer mit kleiner Molmasse enthält, ein entsprechendes Verfahren zur Färbung von Fasern mit diesem Mittel sowie die Verwendung dieses Mittels zur intensiven Färbung keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente so genannte direktziehende Farbstoffe enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Üxidationsfärbemüiei zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Um den Pflegezustand der Fasern zu verbessern, ist es seit langem üblich, die Fasern im Anschluss an die Farbbehandlung einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splissrate verringert.

In jüngster Zeit wurden ferner so genannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird. Dennoch lassen auch diese Mittel, insbesondere auf schwer zu pflegenden Fasern, wie beispielsweise japanischem Haar, noch einige Wünsche hinsichtlich der pflegenden Eigenschaften offen.

Die im Rahmen derartiger Kombinationspräparate einsetzbaren Wirkstoffe müssen insbesondere hinsichtlich ihrer Stabilität hohen Ansprüchen genügen, da die Färbecremes üblicherweise einen hohen pH-Wert aufweisen. Ferner sind Unverträglichkeiten der verschiedenen Wirkstoffe untereinander und somit eine geringe Lagerstabilität zu vermeiden.

Seit einiger Zeit ist es bekannt kationische und/oder amphotere Polymere hoher Molmasse zur Verbesserung des Pflegezustandes keratinischer Fasern auch in oxidativen Färbemitteln einzusetzen. Allerdings vermögen diese Mittel des Standes der Technik noch nicht in jeder Hinsicht zu überzeugen. Sowohl hinsichtlich der Farbintensität der erzielbaren Färbungen als auch hinsichtlich der erzielbaren Strukturstabilisierung bleiben häufig noch Wünsche offen.

Es besteht daher weiterhin ein Bedarf an pflegenden Wirkstoffen für die Färbung von Fasern. Es war somit die Aufgabe der vorliegenden Erfindung ein Färbemittel zur Verfügung zu stellen, das intensive Färbungen bei einer guten Strukturstabilisierung ermöglicht.

Überraschenderweise wurde nunmehr gefunden, dass eine Wirkstoffkombination aus einem Alkyl- und/oder Alkenyl-Oligoglykosid und einem kationischen und/oder amphoteren Polymer mit niedriger Molmasse die oben genannten Nachteile vermeidet.

Ein erster Gegenstand der vorliegenden Anmeldung sind daher Mittel zur Färbung keratinischer Fasern, enthaltend in einem kosmetisch akzeptablen Träger mindestens einen Farbstoff und/oder mindestens ein Farbstoffvorprodukt, sowie weiterhin
(A) mindestens ein Alkyl- und/oder Alkenyl-Oligoglykosid und/oder eines seiner Derivate und
(B) mindestens ein kationisches und/oder amphoteres Polymer mit einer mittleren molaren Masse unterhalb von 20.000.

Unter keratinischen Fasern sind dabei erfindungsgemäß Pelze, Wolle, Federn und insbesondere menschliche Haare zu verstehen.

Zur Bestimmung der erfindungsgemäßen mittleren Molmasse kann die reduzierte Viskosität (dl/g) verwendet werden. Zur Bestimmung der mittleren Molmasse wird im Rahmen der vorliegenden Anmeldung eine 40Gew.-%ige Lösung des jeweiligen Polymers in einer wässrigen 1M-NaCl-Lösung bei pH7 und 30°C mittels eines Ubbelohde-Kapillarviskosimeters vermessen.

Im Rahmen einer ersten bevorzugten Ausführungsform enthalten die erfindungsgemäßen Mittel ein kationisches Polymer mit geringer mittlerer Molmasse.

Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 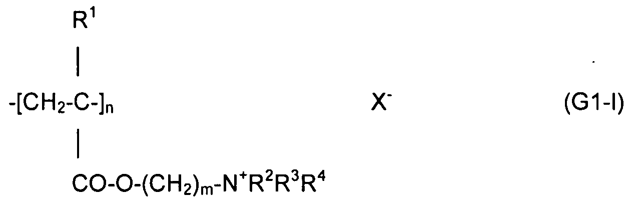 in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nicht-wässrigen Polymerdispersion, die einen Polymeranteil nicht unter 30 Gew.-% aufweisen sollte, eingesetzt. Solche Polymerdispersionen sind mit herkömmlicher Molmassenverteilung unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel mit herkömmlicher Molmassenverteilung erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere mit herkömmlicher Molmassenverteilung, bei denen die Monomere in einem Gewichtsverhältnis von etwa 20:80 vorliegen, sind im Handel als ca. 50 %ige nicht-wässrige Polymerdispersion unter der Bezeichnung Salcare^{®} SC 92 erhältlich.

Weitere bevorzugte kationische Polymere sind beispielsweise
- quaternisierte Cellulose-Derivate, wie sie mit herkömmlicher Molmassenverteilung unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50 mit herkömmlicher Molmassenverteilung,
- kationische Guar-Derivate, wie sie mit herkömmlicher Molmassenverteilung insbesondere unter den Handelsnamen Cosmedia^{®}Guar und Jaguar^{®} vertriebenen Produkte,
- Polysiloxane mit quaternären Gruppen, wie die mit herkömmlicher Molmassenverteilung beispielsweise im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte mit herkömmlicher Molmassenverteilung sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind wie sie mit herkömmlicher Molmassenverteilung unter den Bezeichnungen Gafquat^{®}734 und Gafquat^{®}755 im Handel erhältlich,
- Vinylpyrrolidon-Vinylimidazoliummethochlorid-Copolymere, wie sie wie sie mit herkömmlicher Molmassenverteilung unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie mit herkömmlicher Molmassenverteilung als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise mit herkömmlicher Molmassenverteilung unter den Handelsbezeichnungen Hydagen^{®} CMF, Hydagen^{®} HCMF, Kytamer^{®} PC und Chitolam^{®} NB/101 im Handel verfügbar sind.

Erfindungsgemäß bevorzugte kationische Polymere sind kationische Cellulose-Derivate und Chitosan und dessen Derivate, insbesondere die Handelsprodukte mit herkömmlicher Molmassenverteilung Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Im Rahmen einer bevorzugten Ausführungsform der vorliegenden Erfindung handelt es sich bei den niedermolekularen Polymeren um amphotere Copolymere.

Dabei kann es erfindungsgemäß bevorzugt sein, wenn die kationische Komponenten des Copolymeren ausgewählt ist aus Acrylamidopropyltrimethylammoniumhalogeniden, Methacrylamidopropyltrimethylammoniumhalogeniden, Methyloyloxyethyl trimethylammoniumhalogeniden, Methyloyloxyethyltrimethylammonium Methosulfaten, Acryloyloxyethyltrimethylammoniumhalogeniden und Dimethyldiallylammoniumhalogeniden.

Besonders bevorzugt sind Acrylamidopropyltrimethylammoniumchlorid, Methacrylamidopropyltrimethylammoniumchlorid, Dimethyldiallylammoniumchlorid, Methacryloyloxyethyltrimethylammmoniumchlorid, Methacryloyloxyethyltrimethylammoniummethylsulfat und Acryloyloxyethyltrimethylammoniumchlorid.

Weiterhin kann es erfindungsgemäß bevorzugt sein, wenn die anionische Komponetnen des Copolymeren ausgewählt ist aus Acrylsäure, Methyacrylsäure, 2-Acrylamido-2-methylpropansulfonsäure und 2-Methacrylamido-2-methylpropansulfonsäure. Insbesondere 2-Acrylamido-2-methylpropansulfonsäure kann alleine oder in Kombination mit Acrylsäure oder Methacrylsäure besonders bevorzugt sein.

Das molare Verhältnis der Anteile kationisches Monomer zu anionischem Monomer liegt vorzugsweise zwischen 20 : 80 und 95 : 5.

Als dritten fakultativen Bestandteil kann das amphotere Polymer ein nichtionisches Monomer enthalten. Dies ist vorzugsweise ausgewählt aus C₁-C₂₂ Alkylacrylaten, C₁-C₂₂ Alkylmethacrylate, Acrylamiden, n-Alkylacrylamiden, Methacrylamiden, n-Alkylmethacrylamiden und Diacetonacrylamiden.

Ein erfindungsgemäß bevorzugtes Polymer wird gebildet aus
(a) 1 bis 98 mol % eines kationischen Monomeren ausgewählt aus Acrylamidopropyltrimethylammoniumhalogeniden, Methacrylamidopropyltrimethylammoniumhalogeniden, Methyloyloxyethyl trimethylammoniumhalogeniden, Methyloyloxyethyltrimethylammonium Methosulfaten, Acryloyloxyethyltrimethylammoniumhalogeniden und Dimethyldiallylammoniumhalogeniden;
b) 1 bis 90 mol % eines anionischen Monomeren ausgewählt aus Acrylsäure, Methyacrylsäure, 2-Acrylamido-2-methylpropansulfonsäure und 2-Methacrylamido-2-methylpropansulfonsäure und
(c) 0 bis 80 mol % eines nichtionischen Monomeren ausgewählt aus C₁-C₂₂ Alkylacrylaten, C₁-C₂₂ Alkylmethacrylate, Acrylamiden, n-Alkylacrylamiden, Methacrylamiden, n-Alkylmethacrylamiden und Diacetonacrylamiden.

Erfindungsgemäß besonders bevorzugt ist ein Copolymeres aus Methacrylamidopropyltrimethylammoniumchlorid, Acrylsäure und Methylacrylat wie es beispielsweise unter der INCI-Bezeichnung Polyquaternium-47 und dem Handelsnamen Merquat 2001 von Nalco vertrieben wird.

Weitere erfindungsgemäß einsetzbare pflegende Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten amphoteren Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen niedermolekularen Polymere können mittels herkömmlicher Synthesewege gewonnen werden. Hierzu sei beispielsweise auf die Ausführungen im Rahmen der Beschreibung der WO01/06999 verwiesen.

Das mittlere Molgewicht der erfindungsgemäßen Polymere liegt vorzugsweise unterhalb von 20 000, besonders bevorzugt unter 8 000, ganz besonders besonders bevorzugt unterhalb von 2 000.

Die erfindungsgemäßen Mittel enthalten die kationischen und/oder amphoteren Polymere vorzugsweise in einer Menge von 0,01 bis 5 Gew.-%, insbesondere in einer Menge von 0,1 bis 2 Gew.-%, jeweils bezogen auf die gesamte Anwendungszubereitung.

Als weiteren wesentlichen Bestandteil enthalten die erfindungsgemäßen Mittel mindestens ein Alkyl- und/oder Alkenyl-Oligoglykosid und/oder eines seiner Derivate.

Dabei ist es in einer Ausführungsform der vorliegenden Erfindung bevorzugt, wenn als Alkyl- und/oder Alkenyl-Oligoglykosid (A) eine Verbindung der allgemeinen Formel (I),

R-O-(G)ₚ (I)

mit der Bedeutung
R C₆₋₂₂-Alkylrest oder C₆₋₂₂-Alkenylrest,
G Glykosideinheit, die sich von einem Zucker mit 5 oder 6 Kohlenstoffatomen ableitet,
p Zahl von 1 bis 10
eingesetzt wird.

In den Alkyl- und/oder Alkenylglykosiden der allgemeinen Formel (I) leiten sich die Glykosid-Einheiten G vorzugsweise von Aldosen bzw. Ketosen ab.

Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die besonders bevorzugt eingesetzten Alkyl- und/oder Alkenylgykoside sind daher die Alkyl- und/oder Alkenylglucoside. Alkylglucoside sind erfindungsgemäß ganz besonders bevorzugt.

Die Indexzahl p in der allgemeinen Formel (I) gibt den Oligomerisierungsgrad, d.h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyl- und/oder Alkenylglykosid eine analytische ermittelte rechnerische Größe, die meistens eine gebrochene Zahl darstellt. Vorzugsweise werden Alkyl- und/oder Alkenylglykoside mit einem mittleren Oligomerisierungsgrad p von 1,1 bis 3,0 verwendet. Besonders bevorzugt sind solche Alkyl- und/oder Alkenylglykoside, deren Oligomerisierungsgrad kleiner als 1,5 ist und insbesondere zwischen 1,1, und 1,4 liegt.

Der Alkylrest R leitet sich von primären Alkoholen mit 6 bis 22, vorzugsweise 12 bis 18 Kohlenstoffatomen ab. Typische Beispiele sind Capronalkohol, Caprylalkohol, Caprinalkohol, Laurylalkohol, Myristylalkohol, Cetylalkohol, Stearylalkohol und Behenylakohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.

Neben den genannten Fettalkoholen können sich die Alkylglykoside auch von synthetischen primären Alkoholen mit 6 bis 22 Kohlenstoffatomen, insbesondere den sogenannten Oxoalkoholen ableiten, die einen Anteil von 5 bis 40 Gew.-% verzweigter lsomeren aufweisen.

Besonders bevorzugte Alkylreste sind solche mit 8/10, 12/14, 8 bis 16, 12 bis 16 oder 16 bis 18 C-Atomen. Mischungen der Alkylreste ergeben sich bei einer Herstellung ausgehend von natürlichen Fetten und Ölen oder Mineralölen.

Verfahren zur Herstellung dieser Alkylglykoside sind beispielsweise in den amerikanischen Patentschriften US 3,547,828 und US 3,839,318 sowie der deutschen Patentanmeldung DE-A-37 23 826 beschrieben. Für Alkylpolyglykoside an sich kann beispielsweise auf die DE-A-100 27 975, DE-A-101 38 094 und DE-A-100 31 014 verwiesen werden.

Für eine weitere Diskussion der Alkyloligo/polyglykoside (APG) kann auf WO 03/013450 verwiesen werden.

In einer zweiten bevorzugten Ausführungsform werden Derivate der genannten Alkyl- und/oder Alkenyl-Oligoglykoside eingesetzt.

In diesen Derivaten der Alkyl- und/oder Alkenyl-Oligoglykoside ist vorzugsweise in mindestens einem der Reste G mindestens eine Hydroxylgruppe durch -O-C₁₋₁₂-Alkenyl-COOM, -OSO₃M, -OP(O)(OM)₂ oder -O-CH₂-CH₂-SO₃M mit M H, Alkalimetall, NH₄ ersetzt.

Weitere Hydroxylgruppen können beispielsweise auch verethert sein.

Als derartige Derivate können beispielsweise die Alkyl- und/oder Alkenyl-Oligoglykosidcarboxylate, -sulfate, -phosphate und/oder -isethionate, die sich von den Alkyl- und/oder Alkenyl-Oligoglykosid der allgemeinen Formel (I) ableiten, genannt werden.

Die Herstellung der erfindungsgemäß eingesetzten Alkyl- oder Alkenyl-Oligoglykosidcarboxylate, -phosphate, -sulfate oder -isethionate kann nach bekannten Verfahren erfolgen. Die Herstellung der Carboxylate erfolgt beispielsweise durch Umsetzung der Alkyloligoglykoside mit Salzen von Chlorcarbonsäuren in Gegenwart von Basen. Beispielsweise kann mit 2-Chloressigsäure-Natriumsalz in Gegenwart von NaOH umgesetzt werden. Bei der Umsetzung können sowohl die Hydroxylgruppen im Ring wie auch die -CH₂-OH-Gruppe umgesetzt werden. Der Umsetzungsgrad ist u.a. abhängig von der Stöchiometrie der Einsatzprodukte. Vorzugsweise werden die Alkyloligoglykoside zumindest an der -CH₂-OH-Gruppe umgesetzt, wobei optional ein Mittel eine oder mehreren der am Ring befindlichen Hydroxylgruppen umgesetzt werden können.

Erfindungsgemäß bevorzugt wird ein Alkyloligoglykosid-Carboxylat eingesetzt, in dem -O-C₁₋₁₂-Alkylen-COOM -O(CH₂-)ₙCOOM mit M H, Na oder K und n = 1 bis 3 bedeutet. Besonders bevorzugt ist der Rest O-CH₂-COONa.

Besonders bevorzugt wird ein Alkyloligoglykosidcarboxylat eingesetzt, in dem der Alkylrest ein Laurylrest ist. Speziell bevorzugt ist ein Laurylglucosidcarboxylat, wie es als Plantapon^{®} LGC und Piantapon^{®} LGC sorb von Cognis Deutschland erhältlich ist.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate, sowie Mischungen von Vertretern dieser Gruppen enthalten.

Im Rahmen einer ersten bevorzugten Ausführungsform der vorliegenden Erfindung enthalten die erfindungsgemäßen Mittel mindestens ein Farbstoffvorprodukt vom Entwickler- und/oder Kupplertyp.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 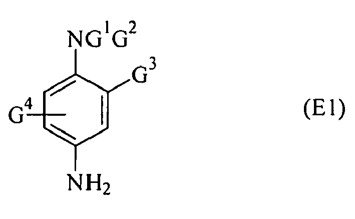 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄-Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 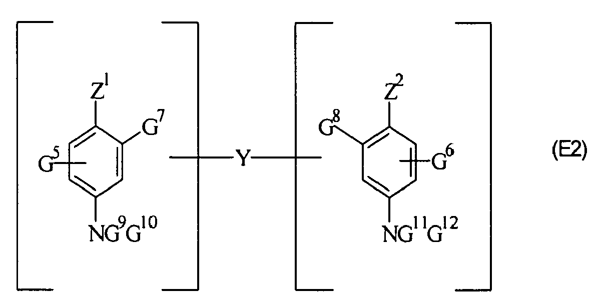 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diaminopropan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiami n, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diaminopropan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 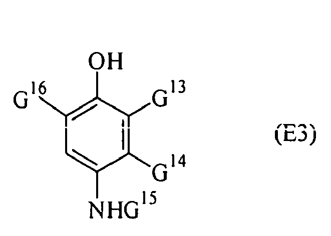 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Dlamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 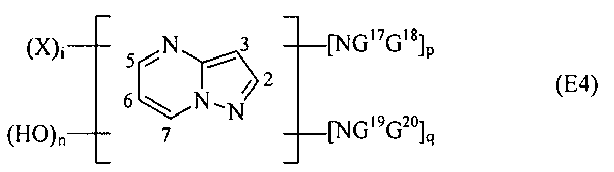 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄- Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

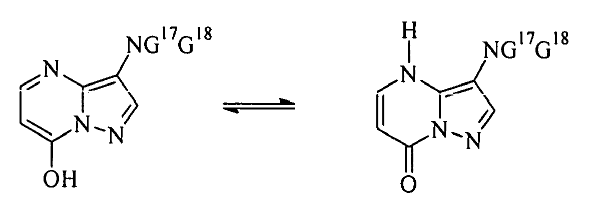

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazoione und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Färbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung enthalten die Färbemittel als Farbstoffvorprodukt (FV) mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIa), 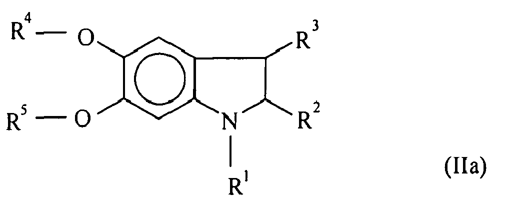 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.
Als Vorstufen naturanaloger Haarfarbstoffe hervorragend geeignet sind weiterhin Derivate des 5,6-Dihydroxyindols der Formel (IIb), 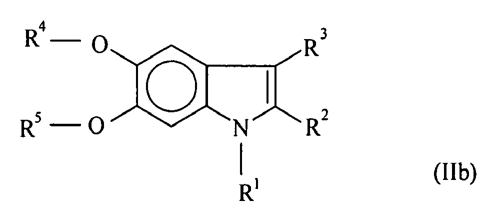 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Im Fall der oxidativen Färbemittel hat es sich als bevorzugt erwiesen, wenn die Färbecreme neben den Farbstoffvorprodukten Silica und/oder Wasserglas enthalten. Insbesondere letzteres wirkt in vielen Fällen korrosionsinhibierend und vermag die Stabilität der Verpackungseinheiten für die erfindungsgemäßen Mittel zu verbessern.

Die erfindungsgemäße Wirkstoffkombination hat sich ferner als besonders geeignet für Färbungen auf Basis von direktziehenden Farbstoffen erwiesen. Neben den beziehungsweise anstelle der erfindungsgemäßen Farbstoffvorprodukte(n) können die erfindungsgemäßen Färbemittel daher in einer weiteren bevorzugten Ausführungsform der vorliegenden Erfindung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahy-drochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

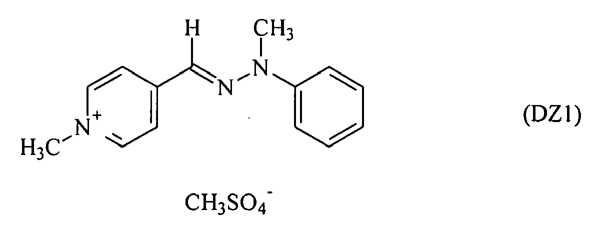

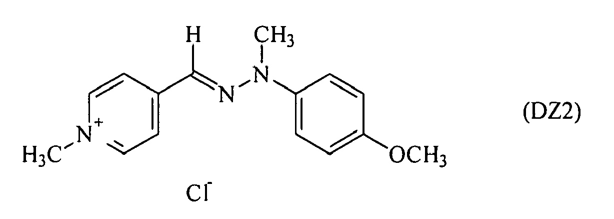

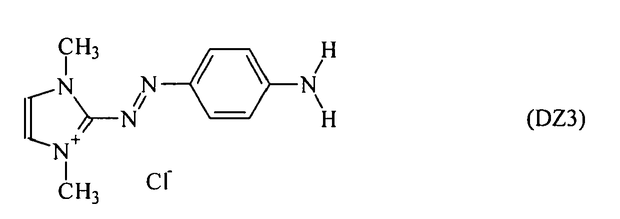

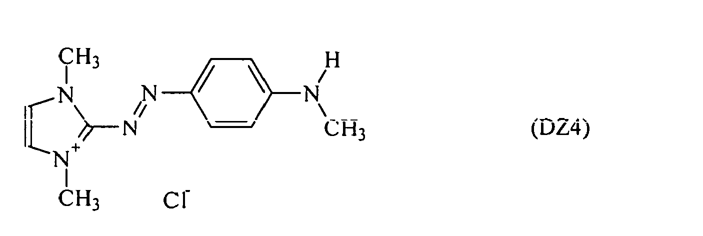

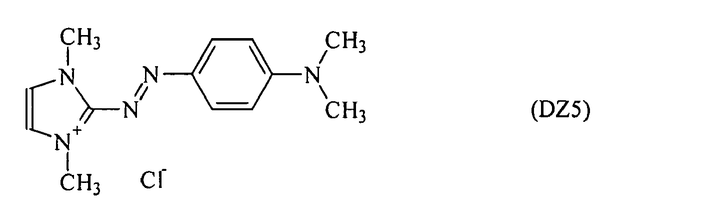

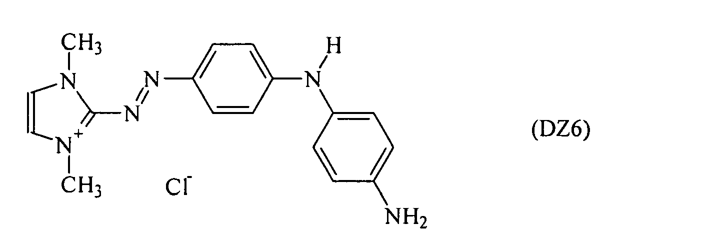

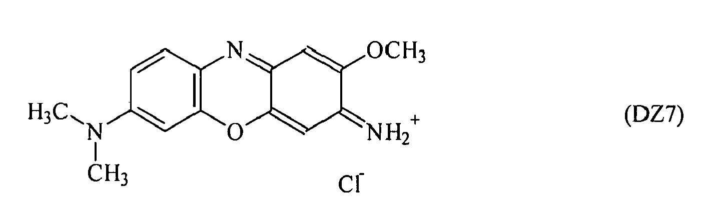

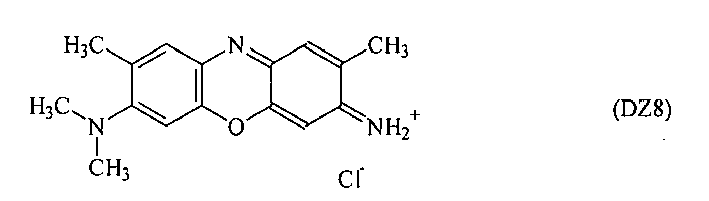

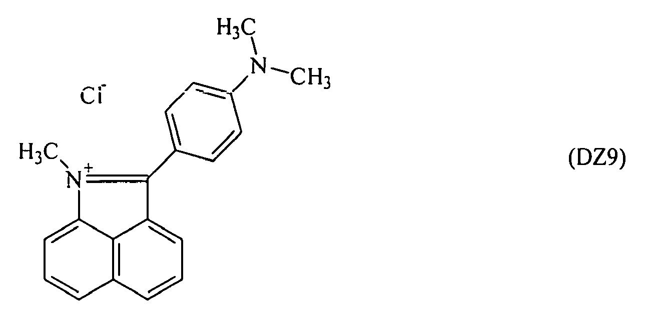

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate.

Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte sowie die erfindungsgemäßen Wirkstoffe, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acryiamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidon, Vinylether und Vinylester.

Bevorzugte anionische Copolymere sind beispielsweise Copolymere aus Acrylsäure, Methacrylsäure oder deren C₁- bis C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁- bis C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Compounds, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnungg Stabileze^{®} QM im Handel erhältlich.

Gemäß einer zweiten Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (I), 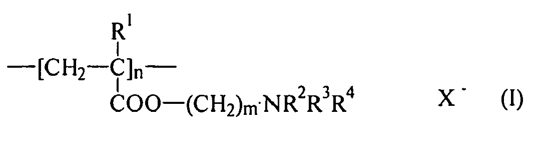 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (I) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Monnitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6) im Handel erhältlich.

Copolymere mit Monomereinheiten gemäß Formel (I) enthalten als nichtionogene Monomereinheiten bevorzugt Acrylamid, Methacrylamid, Acrylsäure-C₁₋₄-Alkylester und Methacrylsäure-C₁₋₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomeren in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

In einer dritten bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben.

Erfindungsgemäß bevorzugte modifizierte Guargums enthalten C₁- bis C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guar Gums sind unter den Handelsbezeichnungen Jaguar^{®} HP8, Jaguar^{®} HP60, Jaguar^{®} HP120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt. Es wird daher explizit auf das Werk von Robert L. Davidson mit dem Titel "Handbook of Water soluble gums and resins", erschienen bei Mc Graw Hill Book Company (1980) verwiesen.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize^{®} der Firma Amerchol und Natrosol^{®} der Firma Hercules vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

Bevorzugt sind weiterhin Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung läßt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁- bis C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze^{®} vertrieben wird.

Aber auch nichtionische, vollsynthetische Polymere, wie beispielsweise Polyvinylalkohol oder Polyvinylpyrrolidon, sind als erfindungsgemäße Verdickungsmittel einsetzbar. Bevorzugte nichtionische, vollsynthetische Polymere werden beispielsweise von der Firma BASF unter dem Handelsnamen Luviskol^{®} vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate als besonders geeignet im Sinne der vorliegenden Erfindung erwiesen. Insbesondere Tone, wie beispielsweise Bentonit, und synthetische Schichtsilikate, wie beispielsweise das von der Firma Süd Chemie unter der Handelsbezeichnung Optigel^{®} vertriebene Magnesiumschichtsilikat, sind bevorzugt.

Weiterhin hat es sich als vorteilhaft erweisen, wenn die Oxidationsmittelzubereitungen mindestens einen Stabilisator enthalten. Besonders bevorzugte Stabilisatoren sind EDTA, 1-Hydroxyethan-1,1-diphoshonsäure, Dipicolinsäure, Phenacetin, Alkalibenzoate (Natriumbenzoat), Alkalistannate (Natriumstannat), Alkalipyrophosphate (Tetranatriumpyrophosphat, Dinatriumpyrophosphat), Salicylsäure, Alkaliphosphate (Natriumphosphat), und Phosphorsäure.

Die Konfektionierung der erfindungsgemäßen Färbemittel unterliegt prinzipiell keinerlei Beschränkungen. Üblicherweise werden die erfindungsgemäßen Mittel als 1-Komponentenmittel konfektioniert, die ggf. unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2- Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der drei erfindungsgemäßen Komponenten separat verpackt wird; es kann aber bevorzugt sein, die Zubereitung (B), die aus Bestandteilen einer Pflanze der Gattung Aloe gewonnen wird, bis zur Anwendung separat zu verpacken.

Weiterhin enthalten die erfindungsgemäßen Mittel in einer bevorzugten Ausführungsform mindestens ein kationisches Tensid.

Erfindungsgemäß bevorzugt sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Eine weitere Gruppe der kationischen Tenside stellen die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen dar. Diese werden beispielsweise von der Firma Mona unter den Handelsbezeichnungen Phospholipid EFA^{®}, Phospholipid PTC^{®} sowie Phospholipid SV^{®} vertrieben.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Die erfindungsgemäßen Mittel können weiterhin alle für solche Zubereitungen bekannten Wirk-, Zusatz- und Hilfsstoffe enthalten. In vielen Fällen enthalten die Färbemittel mindestens ein Tensid. In vielen Fällen hat es sich als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N, N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Ferner können die erfindungsgemäßen Mittel weitere Wirk-, Hilfs- und Zusatzstoffe, wie beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,. Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundiagen und Rezepturen der Kosmetika, 2. Auflage, Hüthig Buch Verlag, Heidelberg, 1989, verwiesen.

Die erfindungsgemäßen Mittel enthalten die Farbstoff(vorprodukt)e sowie die erfindungsgemäßen Wirkstoffe bevorzugt in einem geeigneten wässrigen, alkoholischen oder wässrig-alkoholischen Träger. Zum diesem Zwecke sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbverändernden Mittel in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die folgenden Beispiele sollen den Gegenstand der vorliegenden Anmeldung näher erläutern ohne ihn jedoch in irgendeiner Form zu beschränken.

### 1. Ausführungsbeispiele

Es wurden die folgenden Rezepturen hergestellt. Die Mengenangaben verstehen sich, sofern nichts anderes vermerkt ist, jeweils in Gewichtsprozent.

### 1.1 Versuchsreihe 1

| Rohstoff | Rez. 1 | Rez. 2 | Rez. 3 | Rez. 4 |
|---|---|---|---|---|
| Hydrenol^{®} D | 5,5 | 5,5 | 5,5 | 5,5 |
| Kokoslorol | 2,0 | 2,0 | 2,0 | 2,0 |
| Eutanol^{®} G | 1,0 | 1,0 | 1,0 | 1,0 |
| Eumulgin^{®} B1 | 0,5 | 0,5 | 0,5 | 0,5 |
| Eumulgin^{®} B2 | 0,5 | 0,5 | 0,5 | 0,5 |
| Plantacare^{®} 1200 UP | 2,0 | 2,0 | 2,0 | 2,0 |
| Akypo Soft^{®} 45 NV | 10,0 | 10,0 | 10,0 | 10,0 |
| Texapon^{®} K 14 S 70 C | 2,8 | 2,8 | 2,8 | 2,8 |
| Polymer W37194 | 3,8 | 3,8 | 3,8 | 3,8 |
| Ammoniumsulfat | 0,7 | 0,7 | 0,7 | 0,7 |
| Natriumsulfit | 0,5 | 0,5 | 0,5 | 0,5 |
| Ascorbinsäure | 0,4 | 0,4 | 0,4 | 0,4 |
| p-Toluylendiamin-Sulfat | 0,2 | 0,5 | 0,4 | 0,02 |
| 4-Amino-3-methylphenol | 0,4 | 0,1 | 0,1 | --- |
| 2,4,5,6-Tetraaminopyrimidinsulfat | --- | --- | 1,6 | --- |
| 2-Methylresorcin | --- | --- | 1,0 | --- |
| Resorcin | -- | 0,1 | 0,1 | --- |
| 4-Chlorresorcin | -- | 0,2 | --- | --- |
| 2,7-Dihydroxynaphthalin | 0,2 | --- | --- | --- |
| 5-Amino-2-methylphenol | 0,1 | 0,1 | --- | --- |
| 1-Methoxy-2-amino-4(2-hydroxyethylamino)benzol-Sulfat | -- | --- | --- | 0,01 |
| Rodol 9R | 0,1 | --- | --- | --- |
| Methylgelb | -- | -- | | --- |
| 1,2-Propylenglykol | 0,4 | 0,2 | --- | --- |
| Parfum | 0,3 | 0,3 | 0,3 | 0,3 |
| Turpinal^{®} SL | 0,2 | 0,2 | 0,2 | 0,2 |
| Ammoniak | ad pH 10 | ad pH 10 | ad pH 10 | ad pH 10 |
| Wasser | ad 100 | ad 100 | ad 100 | ad 100 |

Die o. g. Färbecremes 1 - 4 wurden unmittelbar vor der Anwendung mit der folgenden Oxidationsmittelzubereitung vermischt:

| Rohstoff | Menge |
|---|---|
| Dipicolinsäure | 0,6 |
| Natriumpyrophosphat, sauer | 0,03 |
| Turpinal^{®} SL | 1,5 |
| Texapon^{®} N28 | 2,0 |
| Dow Corning^{®} DB 110 A | 0,1 |
| Aculyn^{®} 33 | 12,0 |
| Wasserstoffperoxid (50%ig) | 12,0 |
| Ammoniak | ad pH 4 |
| Wasser | ad 100 |

Zu den resultierenden Anwendungszubereitungen wurden
a) 0 Gew.-% Polyquaternium-47
b) 0,5 Gew.-% Polyquaternium-47
c) 0,75 Gew.-% Polyquaternium-47
zugegeben.

In allen Fällen wurden die Zubereitungen auf die Fasern gegeben und dort 30 Minuten bei Raumtemperatur belassen. Anschließend wurden die Fasern gründlich gespült und getrocknet. Zur Quantifizierung der Farbresultate wurden die Fasern colorimetrisch vermessen; dabei diente jeweils die Versuchsreihe a) als Standard (100% Farbstärke). Es ergaben sich die folgenden Messwerte:

| Rezeptur | Versuch a) | Versuch b) | Versuch c) |
|---|---|---|---|
| Rezeptur 1 | 100% | - | 105,06% |
| Rezeptur 2 | 100% | 110,84% | 106,49% |
| Rezeptur 3 | 100% | 101,59% | 118,02% |
| Rezeptur 4 | 100% | - | 101,86% |

### 1.2 Versuchsreihe 2

| Rohstoff | Rez. 5 | Rez. 6 | Rez. 7 | Rez. 8 | Rez. 9 |
|---|---|---|---|---|---|
| Natrosol | 2,0 | 2,0 | 2,0 | 2,0 | 2,0 |
| Plantacare 1200 | 0,5 | 0,5 | 0,5 | 0,5 | 0,5 |
| Monoethanolamin | ad pH 7 | ad pH 7 | ad pH 7 | ad pH 7 | ad pH 7 |
| 2,4,5,6-Tetraaminopyrimidin-Sulfat | 0,11 | 1,5 | 0,03 | -- | -- |
| p-Toluylendiamin | 0,1 | 0,2 | 0,03 | 1,0 | 1,3 |
| 4-Amino-3-methylphenol | 0,3 | 0,1 | 0,6 | 0,2 | -- |
| 2-Methylresorcin | -- | 0,9 | -- | 0,1 | 0,2 |
| 2,7-Dihydroxynaphthalin | -- | -- | -- | 0,3 | -- |
| m-Aminophenol | 0,1 | -- | -- | 0,1 | 0,1 |
| 4-Chlorresorcin | 0,1 | -- | 0,03 | -- | -- |
| 2-Amino-3-hydroxypyridin | 0,5 | -- | 0,5 | -- | -- |
| Rodol 9R | -- | 0,1 | -- | -- | -- |
| 1-Methoxy-2-amino-4(2-hydroxyethylamino)benzol-Sulfat | -- | -- | -- | -- | 0,4 |
| Resorcin | -- | -- | -- | 0,2 | -- |
| Methylgelb | -- | -- | 0,1 | -- | -- |
| 2,4-Diaminophenoxyethanol-Dihydrochlorid | -- | -- | -- | -- | 0,5 |
| 2-Methylamino-3-amino-6-methoxypyridin | -- | -- | -- | 0,02 | -- |
| Wasser | ad 100 | ad 100 | ad 100 | ad 100 | ad 100 |

Die o. g. Rezepturen 5 - 9 wurden mit
d) 0 Gew.-% Polyquaternium-47
e) 0,5 Gew.-% Polyquaternium-47
versetzt.

In allen Fällen wurden die Zubereitungen ohne weiteren Zusatz eines Oxidationsmittels auf die Fasern gegeben und dort 30 Minuten bei Raumtemperatur belassen. Anschließend wurden die Fasern gründlich gespült und getrocknet. Zur Quantifizierung der Farbresultate wurden die Fasern colorimetrisch vermessen; dabei diente jeweils die Versuchsreihe d) als Standard (100% Farbstärke). Es ergaben sich die folgenden Messwerte:

| Rezeptur | Versuch d) | Versuch e) |
|---|---|---|
| Rezeptur 5 | 100% | 106,5% |
| Rezeptur 6 | 100% | 106,3% |
| Rezeptur 7 | 100% | 111,1% |
| Rezeptur 8 | 100% | 106,6% |
| Rezeptur 9 | 100% | 108,0% |

Beide Messreihen zeigen deutlich die Farbintensivierung (Werte > 100%), die durch den Einsatz der erfindungsgemäßen Wirkstoffkombination erzielt werden kann.

## Patentansprüche

1. Mittel zur Färbung keratinischer Fasern, enthaltend in einem kosmetisch akzeptablen Träger mindestens einen Farbstoff und/oder mindestens ein Farbstoffvorprodukt, **dadurch gekennzeichnet, dass** es weiterhin
(A) mindestens ein Alkyl- und/oder Alkenyl-Oligoglykosid und/oder eines seiner Derivate und
(B) mindestens ein kationisches und/oder amphoteres Polymer mit einer mittleren molaren Masse unterhalb von 20.000
enthält.

2. Mittel nach Anspruch 1, **dadurch gekennzeichnet, dass** das niedrigmolekulare Polymer amphoter ist.

3. Mittel nach Anspruch 2, **dadurch gekennzeichnet, dass** das amphotere Polymer gebildet wird aus
(a) 1 bis 98 mol % eines kationischen Monomeren ausgewählt aus Acrylamidopropyltrimethylammoniumhalogeniden,
Methacrylamidopropyltrimethylammoniumhalogeniden, Methyloyloxyethyl trimethylammoniumhalogeniden, Methyloyloxyethyltrimethylammonium Methosulfaten, Acryloyloxyethyltrimethylammoniumhalogeniden und Dimethyldiallylammoniumhalogeniden;
b) 1 bis 90 mol % eines anionischen Monomeren ausgewählt aus Acrylsäure, Methyacrylsäure, 2-Acrylamido-2-methylpropansulfonsäure und 2-Methacrylamido-2-methylpropansulfonsäure und
(c) 0 bis 80 mol % eines nichtionischen Monomeren ausgewählt aus C₁-C₂₂ Alkylacrylaten, C₁-C₂₂ Alkylmethacrylate, Acrylamiden, n-Alkylacrylamiden, Methacrylamiden, n-Alkylmethacrylamiden und Diacetonacrylamiden.

4. Mittel nach einem der Ansprüche 1 bis 3, **dadurch gekennzeichnet, dass** es als Alkyl- und/oder Alkenyl-Oligoglykosid (A) eine Verbindung der allgemeinen Formel (I),
R-O-(G)ₚ (I)
mit der Bedeutung
R C₆₋₂₂-Alkylrest oder C₆₋₂₂-Alkenylrest,
G Glykosideinheit, die sich von einem Zucker mit 5 oder 6 Kohlenstoffatomen ableitet,
p Zahl von 1 bis 10
enthält.

5. Mittel nach einem der Ansprüche 1 bis 3, **dadurch gekennzeichnet, dass** die Komponente (A) ausgewählt ist aus den Alkyl- und/oder Alkenyl-Oligoglykosidcarboxylaten, -sulfaten, -phosphaten und/oder -isethionaten, die sich von den Alkyl- und/oder Alkenyl-Oligoglykosid der allgemeinen Formel (I) ableiten.

6. Mittel nach einem der Ansprüche 1 bis 5, **dadurch gekennzeichnet, dass** es weiterhin mindestens ein kationisches Tensid enthält.

7. Mittel nach einem der Ansprüche 1 bis 6, **dadurch gekennzeichnet, dass** es als Farbstoffvorprodukt mindestens ein Oxidationsfarbstoffvorprodukt enthält.

8. Mittel nach einem der Ansprüche 1 bis 7, **dadurch gekennzeichnet, dass** es als Farbstoffvorprodukt mindestens ein Indol- und/oder Indolinderivat enthält.

9. Verwendung einer Wirkstoffkombination aus
(A) mindestens einem Alkyl- und/oder Alkenyl-Oligoglykosid und/oder einem seiner Derivate und
(B) mindestens ein kationisches und/oder amphoteres Polymer mit einer mittleren molaren Masse unterhalb von 20.000
zur intensiven Farbveränderung keratinischer Fasern.

10. Verfahren zur Färbung keratinischer Fasern, bei dem eines der Mittel gemäß einem den Ansprüchen 1 bis 8 auf die Fasern aufgetragen wird und nach einer Einwirkzeit wieder abgespült wird.
